# EUROPEAN PATENT APPLICATION

(11) **EP 0 560 227 A2**
(43) Date of publication of application: **15.09.1993**
(21) Application number: 93103565.3
(22) Date of filing: 05.03.1993
(51) Int. Cl.: A61K 35/82, C07G 17/00

(54) **Epstein-barr virus activation inhibitor from lichens**

(30) Priority: 07.03.1992 JP 84688/92
(71) Applicant: Nippon Paint Co., Ltd., Kita-ku, Osaka-shi, Osaka 531 (JP)
(72) Inventor: Miura, Yasutaka, Takatsuki-shi, Osaka-fu (JP); Higuchi, Masako 24-2-506, Ikedakitamachi, Osaka-fu (JP); Kinoshita, Yasuhiro, Osaka-fu (JP); Yamamoto, Yoshikazu, Osaka-fu (JP); Ohigashi, Hajime, Kyoto-shi Kyoto-fu (JP); Koshimizu, Koichi, Nara-shi Nara-ken (JP)
(74) Representative: Hansen, Bernd, Dr. Dipl.-Chem.

(57) **Abstract**

Disclosed is an Epstein-Barr virus activation depressor comprising a component extracted from natural lichens or lichen cultures, and a process for producing the same.

## Description

### FIELD OF THE INVENTION

The present invention relates to a compound derived from natural lichens or lichen cultures, which exhibits antioncogenic promotor activity by inhibiting Epstein-Barr virus activation, and a process for producing the same.

### BACKGROUND OF THE INVENTION

Lichens are consortiums consisting of some kinds of fungi and algae and belong to a group of plants which occupy a botanically specific position. Metabolites of these lichens, that is, lichen components are quite different from components of other various higher or lower plants and they belong to a botanically specific class. They are definitely classified by Asahina et al. [see Asahina and Shibata, "Chemistry of Lichen Components", published by Kawade Shobo, 1948].

It has been considered that a physiological significance of lichen components exists in defense against attack of microorganisms or insect plague because lichens grow slowly, or it exists in defense against ultraviolet rays because they grow in the sunshine, different from other fungi. Therefore, lichen components have hitherto been used for applications which came out of these functions. Examples thereof include dyes, antibiotics, flavors and the like. However, in fact, little study has been made on a pharmacological effect of these lichen components.

Lichens grow slowly and, further, their growth is liable to be restricted by natural environments (e.g. season, climate, temperature, latitude, etc.) as well as artificial environments (e.g. sulphur dioxide gas concentration, smoke concentration, etc.). Therefore, it is extremely difficult to culture lichens, which results in no success. Further, as is often the case in lichens, there the two are similar in form, but totally different in component. Therefore, selection of raw materials requires great skill and it is difficult to collect from nature. In order to obtain a method for producing lichen components, a study on cell culture has recently been made. Since lichens grow rapidly by cell culture in comparison with natural culture which needs a growth period of years or months. Therefore, it is possible to produce a subjective component in a short period of time. Further, different from a natural culture, there are advantages that is is influenced by the weather and need not a lot of persons on collection and, further, it is possible to conduct planned production on a industrial scale. As a method comprising for culturing lichen cultured cells to be extracted and collecting a lichen component from the cells, for example, there is the present inventor's application (Japanese Patent Application No. 58-56689), however, Epstein-Barr virus activation inhibition is not described.

Recently, an assay using Epstein-Barr virus (EBV) activity as an index, which is a kind of a short-term detection method of an oncogenic promotor, has been established by Ito et al. In this assay, by using EBV-EA induced from TPA (12-O-tetradecanoylphorbol-13-acetate) and teleocidin B as an index, it is possible to conduct screening of antioncogenic promotor activity of natural lichens or lichen cultures.

### OBJECTS OF THE INVENTION

One object of the present invention is to provide an extract or active substance having Epstein-Barr virus activation inhibition activity derived form natural lichens or lichen cultures.

Another object of the present invention is to provide a process for producing the same.

These objects as well as other objects and advantages of the present invention will become apparent to those skilled in the art from the following description.

### SUMMARY OF THE INVENTION

According to the present invention, there is provided an Epstein-Barr virus activation inhibitor comprising a component extracted from natural lichens or lichen cultures, and a process for producing the same.

### DETAILED EXPLANATION OF THE INVENTION

The lichen cultures used in the present invention can be derived from any lichens which belong to the following families by a normal method. For example, a method disclosed in Japanese Patent Kokai No. 58-56689 can be employed. Examples of lichens include Teloschistaceae, Physciaceae, Buelliaceae, Usneaceae, Anziaceae, Parmeliaceae, Candelariaceae, Lecanoraceae, Pertusariaceae Acarosporaceae, Umbilicariaceae, Cladoniaceae, Baeomycetaceae, Streocaulaceae, Lecideaceae, Gyalectaceae, Asterothyriaceae, Stictaceae, Peltigeraqceae, Pannariaceae, Coccocarpiaceae, Placynthiaceae, Heppiaceae, Collemataceae, Lichnaceae, Graphidaceae, Thelotremataceae, Diploschistaceae, Verrucariaceae, Pyrenulaceae, Strigulaceae, Sphaerophoraceae, Caliciaceae, Cypheliaceae, Lecanactidaceae, Opegraphaceae, Arthopyreniaceae, Arthoniaceae, Dictyonemataceae, Clavariaceae, Agariaceae and the like. Among them, substances having high Epstein-Barr virus activation inhibition activity can be obtained from lichens which belong to the family Teloschistaceae, Graphidaceae, Parmeliaceae, Usneaceae and Lecideaceae, and they are preferred. The term "lichen culture" used herein means a culture of lichen cells, algae cells or indifferent symbiotic cultured tissue wherein both cells are present.

A culture medium used in the present invention is not specifically limited. Normally, a culture medium which is used for cultures of lichens, or modified culture medium may be used. The culture medium contains microorganic matters such as carbon source, nitrogen source, inorganic ion source, vitamins and the like. Examples of the carbon source include carbohydrates (e.g. sucrose, mannitol, etc.) or derivative thereof. Examples of the nitrogen source include nitrogen-containing compounds such as complicated protein decomposition products (e.g. ammonium ion, nitrate ion, amino acid, peptone, etc.). Examples of the inorganic ion source include inorganic salts (e.g. calcium chloride, magnesium sulfate, etc.) consisting of cations (e.g. potassium ion, calcium ion, magnesium ion, iron ion, etc.) and anions (e.g. sulfate ion, phosphate ion, chloride ion, etc.). Examples of vitamins include thiamin, pyridoxin, nicotinic acid, inositol and the like. As the microorganic matter, phytohormones such as auxins (e.g. 2,4-dichlorophenoxyacetic acid, naphthalene acetic acid, etc.), cytokinins (e.g. kinetin, benzyl adenin, etc.) and the like may be added. As the culture medium used in the present invention, for example, there are modified culture mediums such as Lillie Burnet's culture medium, Hamada's culture medium, malt yeast extract and the like. These culture mediums are used after pH is adjusted to about 6.

The incubation is normally conducted for 3 weeks to 6 months, using above-mentioned solid or liquid culture medium. The incubation temperature varies depending on a kind of lichen cultures, and the incubation is conducted under a constant temperature at 10 to 30°C, preferably 15 to 20°C.

In order to extract an Epstein-Barr virus activation inhibition active substance from natural lichens or lichen cultures, the obtained lichen cultures or natural lichens are directly dipped in a solvent, or dipped in a solvent after they are treated with a drying means (e.g. freeze-drying, etc.). The dipping is conducted at low temperature (0 to 15°C) for 1 to 24 hours, which is due to stability of the Epstein-Barr virus activation inhibition active substance. The solvent may be water, polar organic solvent and non-polar organic solvent. In view of extraction efficiency, polar solvents (e.g. alcohol, ketone, ester, etc.) are preferred (particularly acetone). Then, the dipping solution was filtered off and the solvent of the resulting extract solution was distilled off to obtain an Epstein-Barr virus activation inhibition substance.

The inhibitor of the present invention may be orally used as it is, or used in the form of an oral pharmaceutical composition combined with at least one pharmaceutically acceptable adjuvant. As the dosage form of the oral pharmaceutical composition, for example, there are solid preparations (e.g. tablet, granulate, capsule, etc.) and liquid preparations.

The solid preparation may contains conventional excipients (e.g. silicic anhydride, synthesized aluminum silicate, lactose, cornstarch, crystalline cellulose, etc.), binders (e.g. carboxymethylcellulose, polyvinyl pyrrolidone, etc.), corrigents, sweetners, colorants and the like.

The liquid preparation may be an aqueous or oily suspension, solution, syrup and the like, or it may be formed into a dried article which can be redissolved with a suitable vihicle before use. The liquid preparation may contains normal emulsifiers (e.g. lecithin, sorbitan monooleate, etc.), auxiliary emulsifiers (e.g. sorbit syrup, methylcellulose, gelatin, etc.), non-aqueous vihicles (e.g. coconut oil, peanut oil, etc.), antioxidants, colorants, flavors and the like.

The dosage of the inhibitor of the present invention varies depending on a factor such as constitution of the patient, conditions to be treated and the like. For example, when is is administered to an adult patient, the dosage may be 0.01 to 0.1 g, preferably 0.08 to 0.1 g a day.

The Epstein-Barr virus activation inhibitor comprising a component extracted from natural lichens or lichen cultures of the present invention is extremely useful as an antioncogenic promotor.

The present invention will be further explained in detail in the following examples, but it is not construed to limit to them.

### Example 1

26 Kinds of natural lichens and lichen cultures were freeze-dried and aceton (10 ml) was added to the freeze-dried article (200 mg), followed by extraction at 4°C for twenty-four hours. After filtration, the solvent was distilled off and the extract thus obtained (10 mg) was dissolved in dimethyl sulfoxide (DMSO) to prepare a sample solution (10 µl).

As a culture solution for a FBV latent infected human lymphoid Raji cells, a solution which was prepared by adding a 4 % serum and an antibiotic to RPMI 1640 was used. Raji activity test was conducted as follows. Lactic acid (3 mM) and Teleocidin B (20 ng/ml) were added to Raji cells (density: 5 x 10⁵ cells/ml) which were incubated in a CO₂ incubator at 37°C for 48 hours to make smears. Then, EBV-EA was stained by an indirect fluorescent antibody technique using a serum of a rhinopharyngeal cancer patient. An expression rate of positive cells was defined as 100 % (positive control). According to the same manner as that described above, lactic acid, Teleocidin B and the sample solution (10 µl) were added to Raji cells which were incubated to calculate an expression rate of the sample. Activity of the sample was evaluated by its ratio to 3-ketoursolic acid as the positive control.

Among natural lichens, Alectoria sulcata (2.5), Bryolia fuscesens (1.5), Usnea longissima (4.0), Cetraria ornata (3.1), Pseudoevernia furfuracea (1.9), Umbilicaira kisovana (2.3), Cladonia vulcani (2.8) and Gymnoderma cococarpum (1.7) showed high EBV-EA activity inhibition effect. The numerical value in parenthesis indicates a ratio to 3-ketoursolic acid.

Among lichen cultures, Xanthoria fallax (1.7), Cetraria ornata (2.4), Cladonia vulcani (1.9), Gymnoderma cococarpum (1.8), Rizocarpon sq. (2.0) and Graphis scripta (1.8) showed high EBV-EA activity inhibition effect.

## Claims

1. An Epstein-Barr virus activation inhibitor comprising a component extracted from natural lichens or lichen cultures.

2. The Epstein-Barr virus activation inhibitor according to claim 1, wherein said natural lichens belong to the family Usneaceae, Parmeliaceae and Umbilicariaceae.

3. The Epstein-Barr virus activation inhibitor according to claim 1, wherein said lichen cultures belong to the family Teloschistaceae, Graphidaceae, Parmeliaceae, Usneaceae and Lecideaceae.

4. A process for producing an Epstein-Barr virus activation inhibitor comprising a component extracted from natural lichens or lichen cultures.
